(19) **Europäisches Patentamt**
**European Patent Office**
**Office européen des brevets**

(11) **EP 2 396 113 B1**

(12) **EUROPÄISCHE PATENTSCHRIFT**

(45) Veröffentlichungstag und Bekanntmachung des Hinweises auf die Patenterteilung:
**30.03.2016   Patentblatt 2016/13**

(51) Int Cl.:
**B01L 3/00** (2006.01)     **G01N 33/487** (2006.01)

(21) Anmeldenummer: **10706571.6**

(86) Internationale Anmeldenummer:
**PCT/EP2010/051811**

(22) Anmeldetag: **12.02.2010**

(87) Internationale Veröffentlichungsnummer:
**WO 2010/092160 (19.08.2010 Gazette 2010/33)**

(54) **DIAGNOSTISCHES TESTBAND FÜR FLÜSSIGPROBEN**

DIAGNOSTIC TEST TAPE FOR LIQUID SAMPLES

BANDE DE TEST DIAGNOSTIQUE POUR ÉCHANTILLONS LIQUIDES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **13.02.2009   EP 09152837**

(43) Veröffentlichungstag der Anmeldung:
**21.12.2011   Patentblatt 2011/51**

(60) Teilanmeldung:
**15203140.7**

(73) Patentinhaber:
• **F. Hoffmann-La Roche AG**
  **4070 Basel (CH)**
  Benannte Vertragsstaaten:
  **AT BE BG CH CY CZ DK EE ES FI FR GB GR HR HU IE IS IT LI LT LU LV MC MK MT NL NO PL PT RO SE SI SK SM TR**
• **Roche Diagnostics GmbH**
  **68305 Mannheim (DE)**
  Benannte Vertragsstaaten:
  **DE**

(72) Erfinder:
• **HARTTIG, Herbert**
  **67434 Neustadt (DE)**

• **ROEPER, Josef**
  **67141 Neuhofen (DE)**
• **FUERST, Otto**
  **68519 Viernheim (DE)**
• **JAECK, Thomas**
  **68542 Heddesheim (DE)**
• **DAGENBACH, Ralf**
  **68723 Schwetzingen (DE)**
• **BRAUN, Jürgen**
  **75365 Calw (DE)**
• **MÖNCH, Ronald**
  **68549 Ilvesheim (DE)**
• **LIST, Hans**
  **64754 Hesseneck-Kailbach (DE)**
• **KOSCHORRECK, Beate**
  **69198 Schriesheim (DE)**

(74) Vertreter: **Pfiz, Thomas et al**
  **Wolf Pfiz & Gauss**
  **Patentanwälte**
  **Hauptmannsreute 93**
  **70193 Stuttgart (DE)**

(56) Entgegenhaltungen:
  **EP-A- 0 064 710      EP-A- 0 209 032**
  **EP-A- 0 821 233      EP-A- 1 522 343**
  **EP-A- 1 593 434      EP-A- 1 834 696**
  **DE-B1- 2 118 455     US-A- 5 411 858**

EP 2 396 113 B1

**Beschreibung**

Die Erfindung betrifft ein diagnostisches Testband für Flüssigproben, insbesondere Körperflüssigkeiten, mit einem auf einer Spule aufgewickelten oder aufwickelbaren flexiblen Transportband und einer Vielzahl von auf dem Transportband in Bandlängsrichtung verteilt aufgebrachten Testfeldern, die einen auf das Transportband aufgebrachten Trägerstreifen, eine darauf befindliche Nachweisschicht und ein die Nachweisschicht überspannendes Spreitnetz für eine flächige Flüssigprobenaufnahme umfassen, wobei das Spreitnetz breiter als die Nachweisschicht ist.

Solche Bandsysteme werden vor allem für Blutzuckertests konzipiert, um gegenüber den am Markt befindlichen Teststreifensystemen die Benutzerfreundlichkeit weiter zu verbessern. So kann im Sinne einer vereinfachten Handhabung auf einem aufrollbaren Transportband eine hohe Anzahl von Testeinheiten bzw. Testfeldern kompakt bevorratet und durch den Bandtransport nach Gebrauch auch wieder entsorgt werden. Aus der EP 1 593 434 geht eine einfache Montagetechnik für ein gattungsgemäßes Testband hervor. Damit ist es möglich, eine Rolle-zu-Rolle-Verarbeitung mit hoher Fertigungsgeschwindigkeit zu realisieren. Das als Spreithilfe zur verbesserten Verteilung der Flüssigprobe eingesetzte Gewebe ist dabei prozeßbedingt nur im Bereich seiner überstehenden Längsränder durch eine Klebeschicht gehalten. Löst sich das Gewebe jedoch von der Nachweisschicht, so kann es zu einer ungleichmäßigen Benetzung mit der Blutprobe kommen, wodurch eine korrekte Auswertung erschwert wird. Weiterhin kann es auch vorkommen, dass sich das Spreitgewebe erst nach der Benetzung des Testfeldes mit Blut vom Testfeld löst. Das Blut fließt dann in den noch aufliegenden Gewebebereich und bildet in dem abstehenden Gewebebereich Luftblasen, die einen negativen Einfluss auf die Messauswertung haben.

Ausgehend hiervon liegt der Erfindung die Aufgabe zugrunde, die im Stand der Technik bekannten Erzeugnisse weiter zu entwickeln und einen auch hinsichtlich der Massenfertigung optimierte robusten Bandaufbau für eine zuverlässige Probenverarbeitung anzugeben.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Ein Erfindungsaspekt liegt darin, dass das Spreitnetz im Bereich seiner über die Nachweisschicht überstehenden Seitenränder durch Klebebandstreifen als Abhebesicherung auf dem Trägerstreifen abgestützt und gegen Abheben von der Nachweisschicht gesichert ist, wobei die Klebebandstreifen und die Nachweisschicht eine im Wesentlichen gleiche Dicke aufweisen, so dass das Spreitnetz stufenfrei abgestützt ist.. Hierbei kann auch gewährleistet werden, dass der Abstand zwischen Spreitnetz und Nachweisschicht unter Einsatzbedingungen maximal 40 Mikrometer, vorzugsweise weniger als 20 Mikrometer beträgt, so dass die Gefahr einer ungleichmäßigen Benetzung auch aufgrund von Kapillarkräften weitgehend ausgeschlossen ist.

Eine vorteilhafte Variante sieht vor, dass das rechteckförmige Spreitnetz an seinen Stirnenden jeweils eine quer zur Bandlängsrichtung verlaufende Stoffschlussverbindung, vorzugsweise eine Laserschweißnaht als Abhebesicherung aufweist. Die Laserschweißnaht kann dabei herstellungstechnisch vorteilhaft durch einen Laserschnitt unter Ablängen des Testfelds von einer Rollenware gebildet sein.

In einer weiteren vorteilhaften Ausführung ist das Spreitnetz durch einen um die Nachweisschicht umlaufenden Kleberahmen als Abhebesicherung ringsum auf dem Trägerstreifen fixiert. Zu diesem Zweck kann der Kleberahmen vorteilhafterweise durch Laserverschweißung und/oder Heißklebestellen gebildet sein.

Eine ebenfalls vorteilhafte Ausführung der Abhebesicherung sieht vor, dass das Spreitnetz aus einem gitterartigen Gewebe mit einem in Bandquerrichtung stufenförmig verformbaren biegesteifen Fadensystem besteht. Hierfür ist es günstig, wenn das Gewebe mit Schussfäden aus Metall versehen ist.

Eine Abhebesicherung lässt sich auch dadurch realisieren, dass der Trägerstreifen aus einem robusten Folienmaterial besteht, dessen Scherfestigkeit mehr als 0,05 N/mm2 beträgt und das eine Schälfestigkeit von mehr als 1 N/mm besitzt.

Die Erfindung betrifft auch eine Bandkassette mit einem erfindungsgemäßen diagnostischen Testband, das zur Probenapplikation, vorzugsweise mit einer Bandzugkraft von mehr als 1 N, über eine Umlenkstelle geführt ist.

Im Folgenden wird die Erfindung anhand der in der Zeichnung schematisch dargestellten Ausführungsbeispiele näher erläutert. Es zeigen:

Fig. 1 ein Testband mit einem analytischen Testfeld in einer abgeschnittenen perspektivischen Darstellung;

Fig. 2 einen Querschnitt durch das Testband nach Fig. 1 im Bereich des Testfelds;

Fig. 3 eine Draufsicht auf das Testband nach Fig. 1 im Bereich eines das Testfeld überdeckenden, schräg ausgerichteten Spreitgewebes;

Fig. 4 ein Diagramm der Poissonzahl in Abhängigkeit vom Orientierungswinkel des Spreitgewebes nach Fig. 3;

Fig. 5 bis 9 einen Testbandabschnitt mit verschiedenen Ausführungsformen einer Abhebesicherung für das Spreitgewebe;

Fig. 10      ein Schema der Testfeldfertigung unter Verwendung von Rollenware; und

Fig. 11      eine diagnostische Bandkassette mit einem darin gelagerten Testband in einer aufgebrochenen perspektivischen Darstellung.

Das in der Zeichnung dargestellte diagnostische Testband 10 zur Durchführung von Blutzuckertests umfasst ein aufwickelbares flexibles Transportband 12 und eine Vielzahl von darauf für einen sukzessiven Einmalgebrauch bevorrateten, in Bandlängsrichtung voneinander beabstandeten Testelementen bzw. Testfeldern 14, die als im Umriss rechteckförmige etikettenartige Flächengebilde einen auf das Transportband 12 aufgeklebten Trägerstreifen 16, eine darauf aufgebrachte Nachweisschicht 18 und ein die Nachweisschicht 18 an der von dem Trägerstreifen abgewandten Oberseite überspannendes Spreitnetz 20 für eine flächige Verteilung einer von oben auf das Spreitnetz aufgetragenen Probenflüssigkeit (Blutprobe) umfassen. Die Nachweisschicht 18 spricht als Trockenchemiefilm insbesondere auf Enzymbasis auf einen Analyten (Glucose) durch Farbumschlag an, so dass eine photometrische Erfassung durch den transparenten Folienverbund 12, 16 hindurch erfolgen kann.

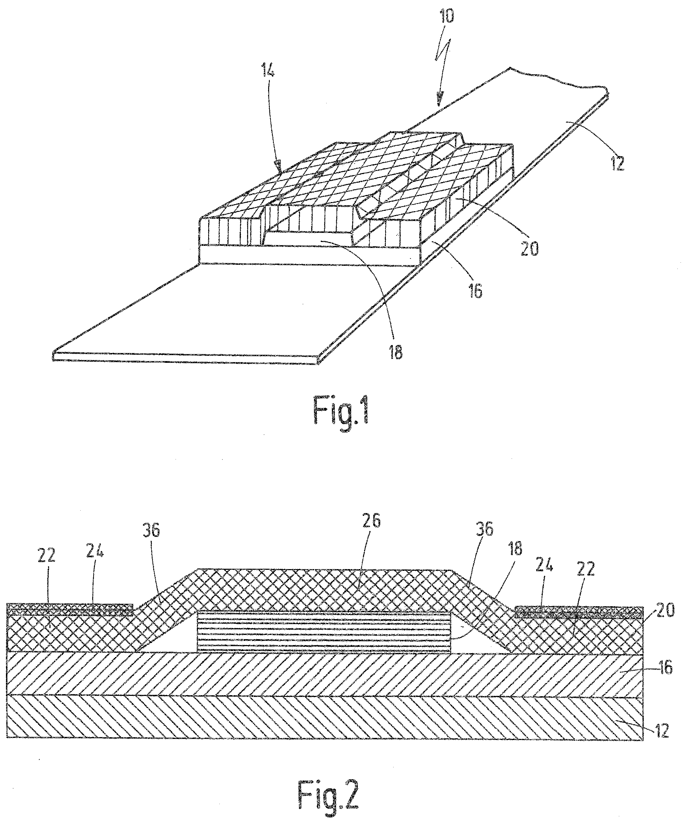

Wie aus Fig. 1 und 2 ersichtlich, ist das als Gewebe 20 ausgebildete streifenförmige Spreitnetz breiter als die Nachweisschicht 18. Die überstehenden Seitenränder 22 des Gewebes 20 sind mit der Oberseite des Trägerstreifens 16 verklebt, der seinerseits als doppelseitiges Klebebandstück auf das Transportband 12 aufgeklebt ist. An ihrer freien Außenseite sind die Seitenränder 22 des Gewebes 20 mit einer hydrophoben Beschichtung 24 versehen, so dass die Flüssigkeitsverteilung bzw. -spreitung gezielt in dem nicht verklebten Zentralbereich 26 des Gewebes 20 über der Nachweisschicht 18 erfolgt.

Die Testfelder 14 lassen sich durch Vorspulen des Transportbandes 12 an einer Applikationsstelle nacheinander zum Einsatz bringen. Aufgrund der dabei ausgeübten Zugkraft ist die flexible Bandstruktur einer Längsdehnung und Querkontraktion unterworfen, was zu einem Abheben bzw. Aufwölben des Zentralbereichs 26 des Gewebes über der Nachweisschicht 18 führen könnte. Dieser Effekt beruht darauf, dass ein in Bandlängsrichtung ausgerichtetes Gewebe durch Bandzugkräfte in seiner Länge, aber nicht in seiner Breite verändert wird, während sich bei dem Trägerband 12 durch Querkontraktion die Breite reduziert.

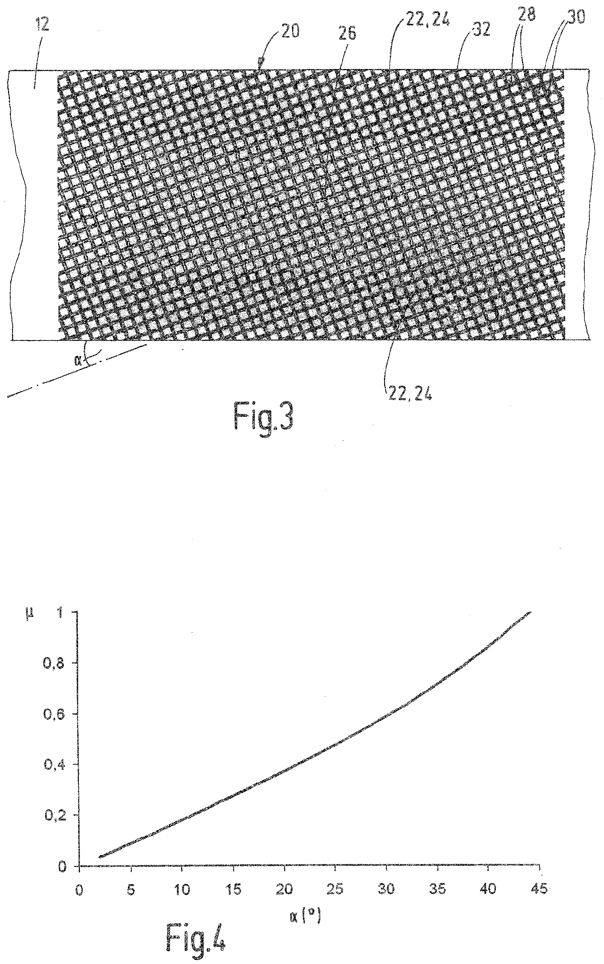

Um diesen für eine gleichmäßige Blutverteilung nachteiligen Abhebeeffekt zu vermeiden, ist das Gewebe 20 schräg zu dem Transportband 12 ausgerichtet, wie es in Fig. 3 veranschaulicht ist. Das Gewebe 20 weist rechtwinklig gekreuzte Gewebefäden 28, 30 auf, die alle schräg zur Bandlängsrichtung bzw. zu den Bandkanten 32 verlaufen.

Zweckmäßig ist das Gewebe 20 in Leinwandbindung aus Kettfäden 28 und Schussfäden 30 gebildet, die in Form von Monofilamenten aus PET bestehen. Für die Verarbeitung von Rollenware ist es günstig, wenn die langen Kettfäden 28 näher zur Bandlängsrichtung als die kurzen Schussfäden 30 verlaufen.

Die Orientierung des Gewebes 20 lässt sich durch einen Kompensationswinkel $\alpha$ angeben, der durch den kleinsten Winkel zwischen einer Bandkante 30 und den Gewebefäden (also in Fig. 3 den Kettfäden 28) definiert ist.

Fig. 4 zeigt die Abhängigkeit der Poissonzahl $\mu$ vom Kompensationswinkel $\alpha$ des Gewebes 20. Allgemein gibt die Poissonzahl die Änderung in der Querdimension d im Verhältnis zur Längsdehnung $\Delta L$ eines Körpers der Länge L nach folgender Beziehung an:

$$\mu = \frac{\Delta d/d}{\Delta L/L}$$

Die Schrägausrichtung des Gewebes 20 wird so bestimmt, dass unter einer vorbestimmten Zugbelastung der Unterschied in der Querkontraktion des Transportbands 12 und des Gewebes 20 minimiert ist. Bei einer gegebenen Poissonzahl des Trägerbandes 12 von etwa 0,4 sollte demnach der Kompensationswinkel im Bereich zwischen 20° und 25° liegen.

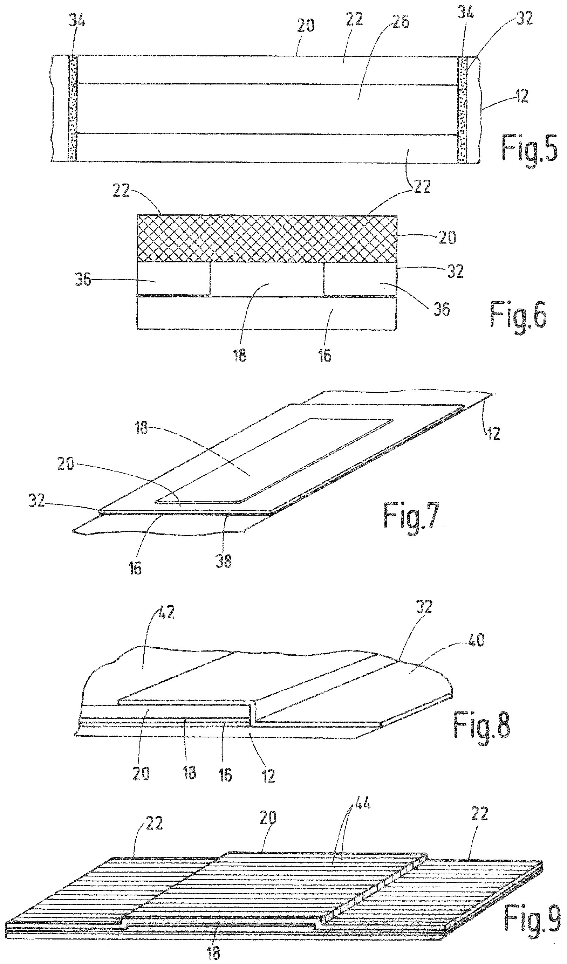

Bei den in Fig. 5 bis 9 gezeigten Ausführungsbeispielen sind gleiche oder ähnliche Teile mit gleichen Bezugszeichen entsprechend der vorstehenden Beschreibung versehen. Das Spreitnetz 20 ist dort anstelle einer Schrägstellung durch eine Abhebesicherung 32 gegen ein Aufwölben bzw. Abheben von der Nachweisschicht 18 gesichert. Diese Abhebesicherung 32 ist bei den verschiedenen Ausführungsbeispielen zusätzlich oder ergänzend zu der seitlichen Verklebung der Seitenränder 22 des Spreitnetzes 20 mit dem Trägerstreifen 16 vorgesehen. Die Ausgestaltung des Spreitnetzes 20 ist hierbei nicht nur auf Gewebe beschränkt, sondern kann auch andere Flachgebilde als Flüssigkeitsverteiler, beispielsweise eine porenhaltige Membrane umfassen. In jedem Fall wird die Abhebesicherung 32 so ausgelegt, dass der Abstand zwischen dem Zentralbereich 26 des Spreitnetzes 20 und der Nachweisschicht 18 unter Einsatzbedingungen des Testbandes maximal 40 $\mu$m, vorzugsweise weniger als 20 $\mu$m beträgt.

Bei der in Fig. 5 gezeigten Ausführungsform ist das rechteckförmige Spreitnetz 20 an seinen in Bandlängsrichtung weisenden Stirnenden jeweils durch eine quer durchgehende Laserschweißnaht 34 gesichert. Diese Fixierung kann durch eine Verschmelzung des Netzmaterials beim Laserschneiden der Testfelder 14 erfolgen, wie es nachstehend näher beschrieben ist.

Eine weitere Verbesserung der Robustheit des Testbands 10 ergibt sich dadurch, dass die in Fig. 2 erkennbare Schräge 36 im Querschnittsverlauf des Spreitnetzes 20 vermieden wird. Dies lässt sich gemäß Fig. 6 dadurch realisieren, dass das Spreitnetz 20 breiter als die Nachweisschicht 18 ist und im Bereich seiner überstehenden Seitenränder 22 durch als Abhebesicherung 32 wirkende Klebebandstreifen 36 auf dem Trägerstreifen 16 seitlich abgestützt ist. Die Klebebandstreifen 36 sollten dabei eine im Wesentlichen gleiche Dicke wie die Nachweisschicht 18 aufweisen, so dass das Spreitnetz 20 plan aufliegt.

Fig. 7 zeigt ein Ausführungsbeispiel, bei dem das Spreitnetz 20 auch stirnseitig über die Nachweisschicht 18 übersteht und an seiner Unterseite durch einen umlaufenden Kleberahmen 38 als Abhebesicherung 32 ringsum auf dem Trägerstreifen 16 fixiert ist. Dies kann durch Laserverschweißung oder eine Kombination von Klebefolien und Laserschweißen erfolgen. Eine Fertigungsmöglichkeit besteht auch darin, eine Klebefolie mit Heißklebepunkten als Zwischenträger in dem Bandfertigungsprozess einzusetzen, um passgenau Klebepunkte an den kurzen und langen Seiten des Kleberahmens 38 zu übertragen.

Bei dem in Fig. 8 gezeigten Ausführungsbeispiel besitzt das Spreitnetz 20 maximal die gleiche Breite wie die Nachweisschicht 18, so dass sich das Netz im entspannten Zustand nicht wölben kann. Alle Komponenten werden dann über seitliche Klebebänder 40 als Abhebesicherung 32 fixiert. Das Klebeband 40 überlappt somit die Längsränder des Schichtaufbaus 16,18,20, wobei ein zentrales Applikationsfenster 42 zum Auftragen der Flüssigprobe freigehalten wird. Möglich ist es auch, dass der das Applikationsfenster 42 bildende Bereich aus einem quer durchgehenden Klebebandstück 40 ausgestanzt wird.

Fig. 9 veranschaulicht ein Ausführungsbeispiel, bei dem das Spreitnetz 20 mit einem in Bandquerrichtung verlaufenden biegesteifen Fadensystem 44 als Abhebesicherung 32 versehen ist. Die Querfäden sind dabei so ausgebildet, dass sie sich weitgehend ohne Luftspalt an den Rand der Nachweisschicht 18 anpressen lassen. Dies kann zweckmäßig dadurch ermöglicht werden, dass ein Gewebe 20 mit Schussfäden aus Metall verwendet wird.

Eine weitere Möglichkeit der Robustheitsverbesserung besteht dadurch, ein geeignetes Folienmaterial für den Trägerstreifen 16 zu wählen. Ein solches Material sollte eine Scherfestigkeit im Bereich von 40N/625mm2 (nach DIN EN 1943) besitzen, während die Schälfestigkeit bei etwa 25N/25mm2 (nach DIN EN 1939) liegen sollte. Derartiges Folienmaterial ist beispielsweise unter dem Handelsname Duplocoll VP20242 erhältlich.

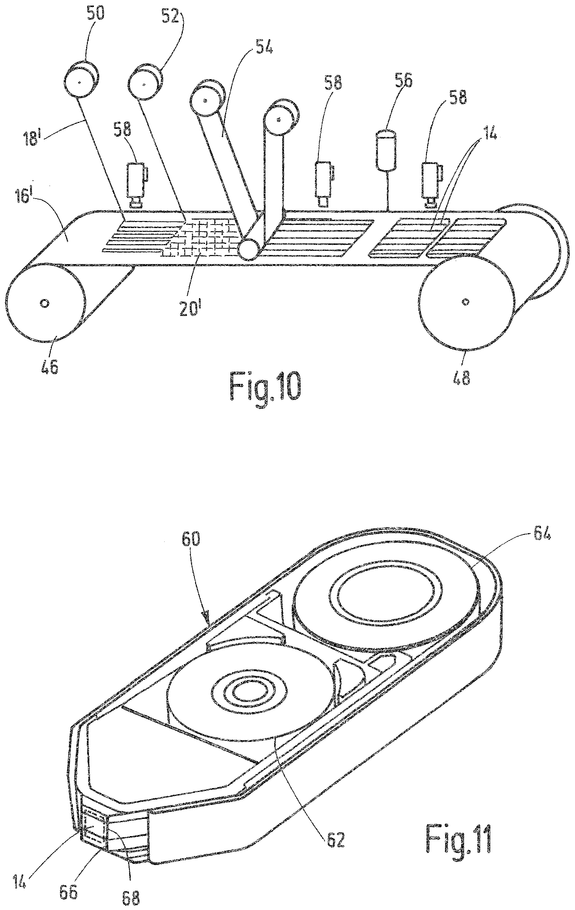

Um eine hohe Fertigungsgeschwindigkeit und -flexiblität bei der Herstellung des Testbandes 10 ermöglichen, ist ein Rolle-Zug-Rolle-Verfahren vorgesehen. Fig. 10 zeigt eine Vorstufe zur mehrspurigen Fertigung der Testfelder 14. Dabei wird ein doppelseitiges Klebeband über eine Fertigungslinie zwischen zwei Rollen 46,48 transportiert. Darauf werden von Spulen 50 mehrere parallele Nachweisfilme 18' aufgebracht. Die Anordnung wird mit Spreitgewebe 20' belegt, das von weiteren Spulen 52 abgezogen wird. Der Schichtaufbau wird anschließend mittels einer Thermotransferfolie 54 fixiert. Sodann werden mit einem Laser 56 quer verlaufende Laserschnitte eingebracht, welche die parallelen Testfelder 14 stirnseitig voneinander trennen. Der Laser 56 kann durch geeignete Wahl der Parameter nicht nur die Testfelder 14 ablängen, sondern auch die Randbereiche zur Bildung der Schweißnaht 34 (Fig. 5) untereinander verkleben. Der gesamte Prozess wird durch Kamerasysteme 58 überwacht, so dass Ausschuss weitgehend vermieden wird. Solchermaßen vorgefertigten Testfelder 14 lassen sich etikettenartig über eine Spendekante auf ein Transportband 12 übertragen und aufkleben, wobei die mehrspurig gefertigten Testbänder 10 dann längsgeteilt werden.

Das Testband 10 wird in Form einer in Fig. 11 gezeigten Bandkassette 60 als Verbrauchsmaterial in ein Handgerät eingesetzt, um es einem Patienten zu ermöglichen, eine Vielzahl von Glucosetests (beispielsweise 50 Tests) jeweils vor Ort selbst durchzuführen. Die Bandkassette 60 weist hierfür eine Abwickelspule 62 zum Abwickeln von ungebrauchtem Testband und eine Aufwickelspule 64 zum Aufwickeln von gebrauchtem Testband auf, wobei das Testband 10 über eine Applikationsspitze 66 gezogen wird, um dort die Testfelder 14 sukzessive für eine Probenaufnahme bereitzustellen. Weitere Einzelheiten der Messwerterfassung ergeben sich beispielsweise aus der EP-A 1 878 379, worauf in diesem Zusammenhang ausdrücklich Bezug genommen wird.

Beim Bandtransport wird das Testband unter einem Bandzug von ca. 4N über die Applikationsspitze 66 geführt. Um hierbei einen ungewollten Abhebeeffekt des Spreitnetzes 20 weiter zu verringern, sind die Radien der Umlenkkanten 68 geeignet abzustimmen. Durch eine Vergrößerung der Radien wird der Stress auf den Mehrkomponentenaufbau 12, 14 und damit die Aufwölbung stark verringert. Jedoch führen zu große Radien zu einer Verbreiterung der Spitze 66, und damit zu einer nachteiligen Vergrößerung der Probenauftragsfläche bzw. des benötigten Probenvolumens. In jedem Fall sollte gewährleistet sein, dass zwischen zwei Umlenkkanten 68 plan gespannt wird.

**Patentansprüche**

1. Bandkassette umfassend ein diagnostisches Testband für Flüssigproben, insbesondere Körperflüssigkeiten, mit einem auf einer Spule (62,64) aufgewickelten oder aufwickelbaren flexiblen Transportband (12) und einer Vielzahl von auf dem Transportband (12) in Bandlängsrichtung verteilten Testfeldern (14), die einen auf das Transportband (12) aufgebrachten Trägerstreifen (16), eine darauf befindliche Nachweisschicht (18) und ein die Nachweisschicht (18) überspannendes Spreitnetz (20) für eine flächige Flüssigprobenaufnahme umfassen, wobei das Spreitnetz (20) breiter als die Nachweisschicht (18) ist, **dadurch gekennzeichnet, dass** das Spreitnetz (20) im Bereich seiner über die Nachweisschicht (18) überstehenden Seitenränder (22) durch Klebebandstreifen (36) als Abhebesicherung (32) auf dem Trägerstreifen (16) abgestützt und gegen Abheben von der Nachweisschicht (18) gesichert ist, wobei die Klebebandstreifen (36) und die Nachweisschicht (18) eine im Wesentlichen gleiche Dicke aufweisen, so dass das Spreitnetz (20) stufenfrei abgestützt ist.

2. Bandkassette nach Anspruch 1, **dadurch gekennzeichnet, dass** die Abhebesicherung (32) so ausgelegt ist, dass der Abstand zwischen Spreitnetz (20) und Nachweisschicht (18) unter Einsatzbedingungen maximal 40 Mikrometer, vorzugsweise weniger als 20 Mikrometer beträgt.

3. Bandkassette nach Anspruch 1, **dadurch gekennzeichnet, dass** das Spreitnetz (20) durch einen um die Nachweisschicht (18) umlaufenden Kleberahmen (38) als Abhebesicherung (32) ringsum auf dem Trägerstreifen (16) fixiert ist.

4. Bandkassette nach einem der Ansprüche 1 bis 3, **dadurch gekennzeichnet, dass** der Trägerstreifen (16) aus einem robusten Folienmaterial besteht, dessen Scherfestigkeit mehr als 0,05 N/mm2 beträgt und das eine Schälfestigkeit von mehr als 1 N/mm besitzt.

## Claims

1. Tape cassette including a diagnostic test tape for liquid samples, in particular body fluids, comprising a flexible transport tape (12) that is wound onto or can be wound onto a spool (62, 64) and a plurality of test fields (14) applied to the transport tape (12) that are distributed in the longitudinal direction of the tape, said test fields comprising a carrier strip (16) applied to the transport tape (12), a detection layer (18) located thereon and a spreading net (20) spanning the detection layer (18) for a two-dimensional uptake of liquid sample, wherein the spreading net (20) is wider than the detection layer (18), **characterized in that** the spreading net (20) is supported in the area of its side edges (22) protruding over the detection layer (18) by strips of adhesive tape (36) as a lifting protection (32) on the carrier strip (16) and is secured against delamination from the detection layer (18), wherein the strips of adhesive tape (36) and the detection layer (18) have essentially the same thickness so that the spreading net (20) is supported without steps.

2. Tape cassette according to claim 1, **characterized in that** the protection against lifting (32) is designed such that the distance between the spreading net (20) and detection layer (18) under the conditions of use is no more than 40 micrometers, preferably less than 20 micrometers.

3. Tape cassette according to claim 1, **characterized in that** the spreading net (20) is attached circumferentially to the carrier strip (16) by an adhesive frame (38) as a protection against lifting (32) which runs all around the detection layer (18).

4. Tape cassette according to one of the claims 1 to 3, **characterized in that** the carrier strip (16) consists of a robust foil material the shear strength of which is more than 0.05 N/mm$^2$ and which has a peel strength of more than 1 N/mm.

## Revendications

1. Cassette à ruban comprenant une bande de test de diagnostic pour des échantillons liquides, en particulier des fluides corporels, avec un ruban de transport (12) flexible enroulé ou pouvant être enroulé sur une bobine (62, 64) et une pluralité de champs de test (14) répartis dans le sens de la longueur de ruban sur le ruban de transport (12), lesdits champs comprenant une bande de support (16) appliquée sur le ruban de transport (12), une couche de mise en évidence (18) se trouvant sur ladite bande de support et un filet de mouillage (20) recouvrant la couche de mise en évidence (18) en vue d'une absorption plate d'échantillon liquide, le filet de mouillage (20) étant plus large que la couche de mise en évidence (18), **caractérisée en ce que** le filet de mouillage (20) est soutenu sur la bande de support (16) dans le secteur de ses bords latéraux (22) surmontant la couche de mise en évidence (18) grâce à des bandes adhésives (36) en tant que sécurité contre le décollement (32) et est ainsi protégé contre un décollement de la couche de mise en évidence (18), les bandes adhésives (36) et la couche de mise en évidence (18) présentant une épaisseur essentiellement identique, de telle manière que le filet de mouillage (20) est soutenu sans changement de niveau.

2. Cassette à ruban selon la revendication 1, **caractérisée en ce que** la sécurité contre le décollement (32) est conçue de telle manière que l'écart entre le filet de mouillage (20) et la couche de mise en évidence (18) est, dans les conditions d'utilisation, inférieur ou égal à 40 micromètres, de manière préférée inférieure à 20 micromètres.

3. Cassette à ruban selon la revendication 1, **caracté-**

**risé en ce que** le filet de mouillage (20) est fixé tout autour de la bande de support (16) en tant que sécurité contre le décollement (32) grâce à un cadre adhésif (38) entourant la couche de mise en évidence (18).

4. Cassette à ruban selon l'une quelconque des revendications 1 à 3, **caractérisée en ce que** la bande de support (16) est constituée d'un matériau en feuille résistant dont la résistance au cisaillement est supérieure à 0,05 N/mm$^2$ et qui possède une résistance au pelage supérieure à 1 N/mm.

Fig.1

Fig.2

Fig.3

Fig.4

Fig.5

Fig.6

Fig.7

Fig.8

Fig.9

Fig.10

Fig.11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1593434 A
- EP 1878379 A